# EUROPÄISCHE PATENTSCHRIFT

(11) **EP 2 310 017 B1**
(45) Veröffentlichungstag und Bekanntmachung des Hinweises auf die Patenterteilung: **15.05.2013**
(21) Anmeldenummer: 09772098.1
(22) Anmeldetag: 20.06.2009
(51) Int. Cl.: A61K 31/541, A61P 33/02

(54) **NIFURTIMOX ZUR BEHANDLUNG VON DURCH TRICHOMONADIDA HERVORGERUFENEN KRANKHEITEN**
NIFURTIMOX FOR TREATING DISEASES CAUSED BY TRICHOMONADIDA
NIFURTIMOX POUR LE TRAITEMENT DE MALADIES PROVOQUÉES PAR LES TRICHOMONADIDA

(30) Priorität: 02.07.2008 DE 102008031283
(43) Veröffentlichungstag der Anmeldung: 20.04.2011
(73) Patentinhaber: Bayer Intellectual Property GmbH, 40789 Monheim (DE)
(72) Erfinder: HARDER, Achim, 51109 Köln (DE); GREIF, Gisela, 53424 Remagen (DE); FROYMAN, Robrecht, 40789 Monheim (DE)
(74) Vertreter: BIP Patents
(86) Internationale Anmeldenummer: PCT/EP2009/004474
(87) Internationale Veröffentlichungsnummer: WO 2010/000398

(56) Entgegenhaltungen:
- EP-A- 0 382 173
- US-A- 3 262 930
- HU J ET AL: "The efficacy of some drugs with known antiprotozoal activity agains Histomonas meleagridis in chickens" VETERINARY PARASITOLOGY, ELSEVIER SCIENCE, AMSTERDAM, NL, Bd. 121, 1. Januar 2004 (2004-01-01), Seiten 233-238, XP002483749 ISSN: 0304-4017
- MCDOUGALD L R: "BLACKHEAD DISEASE (HISTOMONIASIS) IN POULTRY: A CRITICAL REVIEW" AVIAN DISEASES, AMERICAN ASSOCIATION OF AVIAN PATHOLOGISTS, KENNET SQ., PA, US, Bd. 49, Nr. 4, 1. Dezember 2005 (2005-12-01), Seiten 462-476, XP009069336 ISSN: 0005-2086
- RAETHER W ET AL: "Nitroheterocyclic drugs with broad spectrum activity." PARASITOLOGY RESEARCH, Bd. 90, Nr. Supplement 1, Juni 2003 (2003-06), Seiten S19-S39, XP002547457 ISSN: 0932-0113 in der Anmeldung erwähnt
- HARDER A ET AL: "Mechanisms of action of emodepside" PARASITOLOGY RESEARCH ; FOUNDED AS ZEITSCHRIFT FÜR PARASITENKUNDE, SPRINGER, BERLIN, DE, Bd. 97, Nr. 1, 1. Oktober 2005 (2005-10-01), Seiten S1-S10, XP019345740 ISSN: 1432-1955
- MCKELLAR Q A ET AL: "Veterinary anthelmintics: old and new" TRENDS IN PARASITOLOGY, ELSEVIER CURRENT TRENDS, Bd. 20, Nr. 10, 1. Oktober 2004 (2004-10-01), Seiten 456-461, XP004560074 ISSN: 1471-4922
- MONZOTE L: "A review of anti-parasitic patents (1988-2008)" RECENT PATENTS ON ANTI-INFECTIVE DRUG DISCOVERY 2008 GB, Bd. 3, Nr. 3, 2008, Seiten 177-191, XP009123144 ISSN: 1574-891X

## Beschreibung

Die vorliegende Erfindung betrifft die Verwendung von Nifurtimox zur Behandlung von durch Trichomonadida verursachten Krankheiten, wie z. B. der Histomoniasis, insbesondere bei Puten.

Die Wirksamkeit von nitroheterocyclischen Verbindungen gegen Protozoen-Erkrankungen ist im Prinzip bekannt (1)

Zu den Protozoen zählen einkernige Organismen, deren Grundstruktur eine eukaryontische Zelle ist. In der genauen Systematik gibt es jedoch große Unterschiede in der Lebensweise, Morphologie und dem biochemischen Stoffwechsel der einzelnen Stämme, Klassen, Gattungen und Arten. Daher wirken chemische Substanzen je nach ihrem Angriffspunkt und Wirkprinzip üblicherweise nicht gleichermaßen gegen alle Protozoen sondern nur gegen spezielle Gruppen von Protozoen (2, 3, 4).

Bisher ist die Wirksamkeit von Nifurtimox nur gegen Protozoen-Arten der Gattung Trypanosoma beschrieben worden, z. B. *Trypanosoma brucei* und *Trypanosoma cruzi* (5, DE-AS-1 170 957). Trypanosomen besitzen eine Geißel, die am Basalkörper ("Kinetosom", daher Ordnung der Kinetoplastida) entspringt und in Verbindung mit dem Grundkörper eine undulierende Membran ausbildet. Trypanosomen vermehren sich überwiegend im Blutplasma und werden durch blutsaugende Arthropoden übertragen. Diese Erreger verursachen die Chagas-Erkrankung ("Trypanosomiasis") des Menschen. Nifurtimox ist derzeit fast die einzige Verbindung, die gegen diese Erreger wirkt. Diese Wirksamkeit beruht vermutlich auf der Hemmung der Trypanothione Reduktase, einem speziellen Enzym von Trypanosomen. Dieses Enzym fehlt anderen Protozoen-Erregern, insbesondere Trichomonaden und Histomonaden.

Innerhalb der Ordnung der Trichomonadida und Diplomonadida ist die Wirksamkeit von Nifurtimox bisher nicht beschrieben worden. Trichomonadida sind durchweg parasitisch lebende Protozoen für die mehrere in dere Regel 4 bis 6 Flagellen typisch sind. Eine große morphologische Besonderheit ist ein kontraktiler Stab (= Costa) im Inneren der Organismen, welcher an der Bewegung beteiligt ist. Im Gegensatz zu den Kinetoplastiden besitzen Trichomonadida keine Mitochondrien, das sind wichtige Organellen des Energiestoffwechsels. Der Energiestoffwechsel erfolgt stattdessen in sogenannten Hydrogenosomen. In diesen Organellen ist die oxidative Decarboxylierung von Pyruvat an die ATP Synthese und an einen Ferredoxin gesteuerten Elektronen-Transport gekoppelt (6). Die Vermehrung dieser einzelligen Parasiten erfolgt durch Zweiteilung. Es treten keine sexuellen Stadien oder Zysten auf. Zur Ordnung der Trichomonadida gehören viele Gattungen (insbesondere die Gattungen Trichomonas und Histomonas) und desweiteren viele Arten, jedoch sind die meisten davon eher harmlos und apathogen. Dennoch gibt es Vertreter, die schwerwiegende Erkrankungen auslösen und in der Tierhaltung bedeutende wirtschaftliche Schäden verursachen. Hierzu zählt die Gattung Trichomonas (insbesondere *T*. *gallinae* und *T*. *gallinarum*)*,* Die Gattung Tritrichomonas (insbesondere *T*. *foetus* und *T*. *suis*) und die Gattung Histomonas (insbesondere *H. meleagridis*)*.*

Bei der Trichomonose der Taube und des Haushuhns handelt es sich um eine Infektionskrankheit, die durch *Trichomonas gallinae* und *T*. *gallinarum* hervorgerufen wird. *T*. *gallinae* parasitiert primär im Rachen, in der Speiseröhre und im Kropf. Im Verlauf der Krankheit werden aber auch andere Organe, vor allem die Leber, das Herz und die Lunge befallen. Die Infektion junger Tauben erfolgt bereits bei der ersten Fütterung mit Kropfmilch von latent infizierten älteren Tieren. Weitere Infektionsquellen sind verseuchtes Trinkwasser oder Futter. Die Erkrankung gilt als häufigste Jungtiererkrankung bei Tauben und richtet besonders in Zuchtbeständen große Schäden an. Neben der hohen Mortalität treten Verdaungsstörungen, Appetitlosigkeit, eine behinderte Trinkwasser - und Nahrungsaufnahme sowie ein eingeschränktes Flugvermögen auf. *Trichomonas gallinarum* parasitiert im Blinddarm von Huhn und Pute. Die Erkrankung verursacht Wachstumsverzögerungen, schwerwiegende Durchfälle sowie eine nekrotische Entzündung der Leber.

Bei der Histomoniasis handelt es sich um eine Infektionskrankheit, die durch *Histomonas meleagridis* hervorgerufen wird. Histomonaden sind Darmparasiten. Besondere Bedeutung hat die Histomoniasis bei Puten, man spricht in diesem Fall auch von der sogenannten Schwarzkopfkrankheit. Bei Puten wird die Krankheit insbesondere durch den Erreger *Histomonas meleagridis* verursacht. Neben der Pute können auch Hühner, Perlhühner, Pfaue, Fasane, Rebhühner und Wachteln an dem Erreger erkranken und gelten als Reservoirwirte.

Durch die *Histomonas meleagridis-*Infektion kommt es zu einer schweren Blinddarm- und Leber-Entzündung, da der Erreger das Darmgewebe schädigt und über das Blut in die Leber gelangt, wo er eine Nekrosebildung bewirkt. Als Begleiterscheinung der Krankheit tritt oft Kreislaufversagen auf, kenntlich an den schwarz-blauen Köpfen erkrankter Tiere, denen die Krankheit ihren Namen verdankt.

In infizierten Tierbeständen, z. B. in Geflügelzuchtbetrieben breitet sich die Erkrankung sehr schnell auf den gesamten Bestand aus und führt durch sehr hohe Mortalitätsraten (unter Umständen bis zu 100%) zu großen wirtschaftlichen Verlusten.

*Histomonas meleagridis* gehört aufgrund seiner strukturellen Geißeln zum Unterstamm der Flagellaten (Mastigophora) und zur Ordnung der Trichomonadida. Die begeißelten Stadien vermehren sich im Blinddarm durch Zweiteilung. Amöboidartige Stadien dringen über die Blutbahn ausgehend vom infizierten Blinddarm in die Leber ein und zerstören diese über großflächige Nekrosen (7)

Die Übertragung der Histomonaden auf direktem Weg, z.B. die orale Aufnahme von histomonadenhaltigem, frischem Kot, ist selten, da die Erreger außerhalb eines Wirtes nur für kurze Zeit lebensfähig sind und bei der Passage des Verdauungstraktes in den meisten Fällen abgetötet werden. Tests amerikanischer Forscher ergaben, dass im Tierversuch eine Infektion bei Puten über die Kloake wesentlich leichter erfolgt als auf oralem Weg. Da die Kloake nach dem Kotabsetzen einen leichten Sog erzeugt, ist eine Infektion auf diesem Weg unter praktischen Bedingungen, z.B. über verschmutzte Einstreu, wahrscheinlich. Wissenschaftlich eindeutig nachgewiesen ist die Erregerübertragung über Zwischenwirte. Als Überträger (insbesondere als Transportvektor von *Histomonas meleagridis*) ist vor allem der Blinddarmwurm *Heterakis gallinarum* (Eier oder Larven) bekannt. In embryonierten Heterakis-Eiern können Histomonaden bis zu 4 Jahren infektiös bleiben. Weitere Zwischenwirte können Regenwürmer und Arthropoden sein, die mit Heterakis-Eiern kontaminiert sind. Auch Hühner und andere Geflügelarten stellen eine potentielle Gefahr dar. Sie sind weniger empfindlich als Puten und tragen oftmals den Erreger in sich, ohne klinisch auffällig zu sein; so tragen sie zur Verbreitung des Erregers bei.

Puten können in jedem Lebensalter infiziert werden, am häufigsten tritt die Erkrankung jedoch zwischen der 3. und 12. Lebenswoche auf. Der Zeitraum zwischen Infektion und Krankheitsausbruch beträgt zumeist 7-12 Tage. Die Sterblichkeit kann bis zu 100% betragen und erreicht ihren höchsten Punkt am 17. Tag nach der Infektion. Ab dem 8. Tag sind Entzündungen im Blinddarm, ab dem 10. Tag in der Leber zu finden.

Infizierte Tiere sind matt und erschöpft, lassen Flügel und Köpfe hängen und verweigern die Futteraufnahme. Typisch ist das Absetzten von schwefelgelbem Kot, Durchfall und später auch Blutbeimischungen. Die mit der Krankheit einhergehenden Kreislaufstörungen verursachen eine ausgeprägte schwarz-blaue Färbung des Kopfes, die der Krankheit ihren Name gab.

Der Verlauf der Erkrankung wird in erster Linie vom Alter und der Darmflora der Puten bestimmt. Zusätzliche Bakterieninfektionen mit E. *coli, Clostridium perfringens* oder Coccidien können den Verlauf erschweren.(8)

Die Diagnose der Histomoniasis kann über Nativpräparate aus Blinddarm und Leber mit Hilfe einer Kochsalzlösung erfolgen. Amöboid bewegliche Stadien sind im Phasenkontrastmikroskop sichtbar. Histologisch ist die PAS-Färbung im Gebrauch.

Bis 1950 waren Arsen-Verbindungen (z.B. Nitarson, Carbarson, Roxarson) die einzig wirksamen Verbindungen gegen Histomoniasis. Jedoch ist bekannt, dass Arsenverbindungen generell nicht stark genug sind, einmal etablierte Infektionen zu behandeln. Ein weiterer Nachteil ist ihr extrem geringer Sicherheitsindex: bereits eine 2fache Dosierung von Roxarson führt zu gestörten motorischen Funktionen im Truthahn.

Seit 1960 wurden andere Nitroimidazole oder Nitrofurane z. B. im Futter oder im Trinkwasser eingesetzt, jedoch Mitte der 90er Jahre zunehmend von der EU und den USA für die Anwendung im Nutztier und als Futterzusatzstoff verboten: Dimetridazol wurde 1997 in USA vom Markt genommen und 2001 für die Nutzung als Futterzusatzstoff in der EU verboten. Seit dem 31.03.2003 darf nunmehr auch Nifursol, das einzige in der EU noch zugelassene Produkt, wegen Sicherheitsbedenken nicht mehr eingesetzt werden. Somit stehen derzeit und in Zukunft weder Arzneimittel für die Therapie noch Präparate zur prophylaktischen Bekämpfung der Histomoniasis zur Verfügung.

Die derzeit einzig verfügbaren Strategien zur Vermeidung einer Erkrankung bestehen aus Maßnahmen zur Hygiene, Optimierung der Haltungsdichte und der Nährstoffversorgung sowie dem Vermeiden einer Erregerverschleppung. Diese Maßnahmen sind unzureichend und können eine Infektion und Erkrankung alleine nicht verhindern.

Impfstoffe gegen die Histomoniasis stehen nicht zur Verfügung. Beispielsweise ist eine Impfung gegen *Histomonas meleagridis* biologisch nicht möglich, da auch eine natürliche Immunität nach einer Infektion nicht erworben werden kann. Einmal infizierte Tiere können erneut erkranken. Versuche über attenuierte Lebendimpfstoffe zu immunisieren waren erfolglos.

Es besteht daher ein Bedarf an Wirkstoffen zur Behandlung von durch Trichomonadida hervorgerufenen Krankheiten, wie z. B. der Histomoniasis, die gute Wirkung und gute toxikologische Eigenschaften aufweisen.

Überraschenderweise haben wir jetzt die Wirksamkeit von Nifurtimox gegen Trichomonadida gefunden, wobei Nifurtimox auch gute toxikologische Eigenschaften aufweist. Diese Wirksamkeit ist bisher nicht beschrieben worden und auch die guten toxikologischen Eigenschaften waren nicht erwartet worden.

Die Erfindung betrifft:
Die Verwendung von Nifurtimox zur Herstellung von Arzneimitteln zur Behandlung von durch Trichomonadida hervorgerufenen Krankheiten.

Nifurtimox ist die Verbindung der Formel (I):

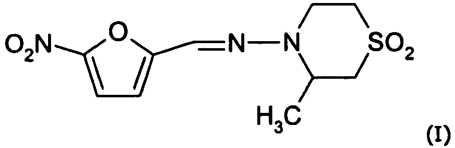

Gegebenenfalls kommt auch der Einsatz in Form üblicher pharmazeutisch verträglicher Salze in Frage. Weiterhin kommt gegebenenfalls auch der Einsatz von Hydraten oder anderen Solvaten der Wirkstoffe oder gegebenenfalls ihrer Salze in Frage.

Die Anwendung kann sowohl prophylaktisch als auch therapeutisch erfolgen. Zu den Trichomonadida zählen die Gattungen Histomonas, Trichomonas, Tritrichomonas. Aus der Gattung Trichomonas seien insbesondere genannt *T*. *gallinae* und *T*. *gallinarum.* Aus der Gattung Tritrichomonas seien insbesondere genannt *T*. *foetus, T*. *suis* und *T*. *equii.* Aus der Gattung Histomonas sei insbesondere *H. meleagridis* genannnt.

Zu den Dipomonadida zählt die Gattung Hexamita. Aus der Gattung Hexamita seine insbesondere genannt *H. columbae, H. meleagridis* und *H. salmonis.*

Bevorzugt ist die Bekämpfung der Histomoniasis. Sie wird hervorgerufen durch Histomonas spp.. Ganz besonders bevorzugt ist die erfindungsgemäße Bekämpfung der Histomoniasis hervorgerufen durch *Histomonas meleagridis.* Die Wirksamkeit von Nifurtimox bei der Bekämpfung der Histomoniasis richtet sich nicht nur gegen die Erregerstadien des Darmes sondern auch gegen die Leberstadien der Erreger.

Erfindungsgemäß behandelt werden Tiere. Beispielsweise seien Säugetiere wie z. B. Rind, Pferd, Schwein, Hund, Katze genannt. Vorzugsweise behandelt wird Geflügel, wie z. B. Hühner, Perlhühner, Rebhühner, Wachteln, Enten, Gänse, Pfaue, Fasane, Tauben und insbesondere Puten (hier synonym mit Truthähnen).

Beispielhaft hervorgehoben seien folgende Krankheiten:
Trichomonose der Taube, der Pute oder des Haushuhns, hervorgerufen durch *T*. *gallinarum* und/oder *T*. *gallinae.*

Histomoniasis bei Hühnern, Perlhühnern, Pfauen, Fasanen, Rebhühnern, Wachteln und insbesondere bei der Pute. Die Histomoniasis bei Puten (Schwarzkopfkrankheit) wird insbesondere durch *H. meleagridis* hervorgerufen.

Die Anwendung der Wirkstoffe erfolgt direkt oder in Form von geeigneten Zubereitungen enteral, parenteral, dermal.

Die enterale Anwendung der Wirkstoffe geschieht z.B. oral in Form von Pulver, Zäpfchen, Tabletten, Kapseln, Pasten, Tränken, Granulaten, Drenchen, Boli, medikiertem Futter oder Trinkwasser. Die dermale Anwendung geschieht z.B. in Form des Tauchens (Dippen), Sprühens (Sprayen), Badens, Waschens, Aufgießens (pour-on and spot-on) und des Einpuderns. Die parenterale Anwendung geschieht z.B. in Form der Injektion (intramusculär, subcutan, intravenös, intraperitoneal) oder durch Implantate.

Geeignete Zubereitungen sind:
Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;
Emulsionen und Suspensionen zur oralen oder dermalen Anwendung sowie zur Injektion; Halbfeste Zubereitungen;
Formulierungen, bei denen der Wirkstoff in einer Salbengrundlage oder in einer Öl in Wasser oder Wasser in Öl Emulsionsgrundlage verarbeitet ist;
Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden z. B. intravenös, intramuskulär und subcutan verabreicht.

Orale Lösungen werden direkt angewendet. Konzentrate werden nach vorheriger Verdünnung auf die Anwendungskonzentration oral angewendet.

Lösungen zum Gebrauch auf der Haut werden aufgeträufelt, aufgestrichen, eingerieben, aufgespritzt, aufgesprüht oder durch Tauchen (Dippen), Baden oder Waschen aufgebracht.

Gele werden auf die Haut aufgetragen oder aufgestrichen oder in Körperhöhlen eingebracht.

Aufgießformulierungen werden auf begrenzte Bereiche der Haut aufgegossen oder aufgespritzt, wobei der Wirkstoff entweder die Haut durchdringt und systemisch wirkt oder sich auf der Körperoberfläche verteilt.

Emulsionen sind entweder vom Typ Wasser in Öl oder vom Typ Öl in Wasser und können oral, dermal oder als Injektionen angewendet werden.

Suspensionen können oral, dermal oder als Injektion angewendet werden.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen werden die Wirkstoffe mit geeigneten Trägerstoffen gegebenenfalls unter Zusatz von Hilfsstoffen vermischt und in die gewünschte Form gebracht.

Erfindungsgemäß besonders bevorzugt ist die Anwendung bei Geflügel. Diese erfolgt vorzugsweise oral, inbesondere durch medikiertes Futter oder über das Trinkwasser.

Alle oben genannten Arzneimittelformen, die zu verwendenden Zusatz- und Hilfsstoffe sowie die Herstellung dieser Arzneiformen sind dem Fachmann grundsätzlich bekannt.

Die Wirkstoffe können in Kombination mit Synergisten oder mit weiteren Wirkstoffen vorliegen. Als weitere Wirkstoffe seien genannt:
Coccidiostatika, wie Robenidin oder Amprolium, z.T. in Kombination mit Folsäureantagonisten (z. B. Ethopabate, Pyrimethamine, Epiroprim);
Polyetherantibiotika wie Monensin, Salinomycin, Lasalocid, Narasin, Semduramicin oder Maduramicin;
Triazinone, wie Toltrazuril, Ponazuril oder Diclazuril;
Sulfonamide (Sulfaquinoxalin, Sulphadimidin, Sulfadiazin);

Für einen langfristigen Behandlungseffekt ist es zu empfehlen, die Tierhaltungen vor der Einstallung oder nach Abschluß der Mastperiode zu desinfizieren.

Helminthen, insbesondere *Heterakis spp. (Larven) (9)* fungieren als Transportvektoren bei der Übertragung der Histomonaden. Bei der Behandlung der Histomoniasis kann daher eine Kombinationsbehandlung mit Anthelmintika sinnvoll sein. So ist bekannt, dass Anthelminthika wie z. B. die Benzimidazole Albendazol oder Fenbendazol in-vivo prophylaktisch wirksam sind, wenn ab der Infektion behandelt wird. Üblich ist eine anthelmintische Behandlung 14 Tage lang ab dem Infektionszeitpunkt. Durch eine kombinierte Behandlung mit Nifurtimox und Anthelmintika kann daher eine verbesserte Behandlung von durch Trichomonaden hervorgerufenen Krankheiten erreicht werden.

Als Anthelmintika seien Benzimidazole wie Albendazol, Fenbendazol oder Probenzimidazole wie Febantel genannt. Diese Substanzen sind z. B. wirksam gegen *Heterakis spp.,* insbesonderes gegen *Heterakis gallinarum,* der bekanntlich als Transportvektor von *Histomonas meleagridis* fungiert (10).

Desweiteren genannt seien Imidazolthiazole (Levamisol, Tetramisol), Tetrahydropyrimidine (Pyrantel, Morantel, Oxantel), Amidin-Derivate z.B. Amidantel, Tribendimidin, sowie das deacylierte Amidantel-Derivat Bay d 9216 und Aminoacetonitril-Derivate (siehe z. B. Kaminsky et al., Nature 452, 176-180 (13 March 2008), wie z. B. AAD 1470.

Bevorzugt unter den Anthelminthika seien Depsipeptid-Anthelminthika genannt. Depsipeptid-Anthelminthika wie das PF1022A und das Emodepsid haben eine breite anthelminthische Wirksamkeit gegen Nematoden in verschiedenen Tierarten wie Hühnervögel, Nagetiere, Reptilien, Hunde, Katzen, Schafe, Rinder, Ziegen, Pferde (11, 12, 13, 14). Dabei konnte gezeigt werden, dass PF1022A und Emodepsid gegen Nematoden der Überfamilie der Heterakoidea wirksam ist. Zu diesen gehört z. B. aus der Familie der Heterakidae neben *Heterakis gallinarum* im Huhn der Mausnematode *Heterakis spumosa.* Gegen letzteren ist PF1022A z. B. wirksam bei einer oralen Dosis von 50 mg/kg sowie Emodepsid z. B. im Dosisbereich von 1-10 mg/kg (13, 15). Weiterhin ist z. B. PF1022A nachgewiesenermaßen wirksam gegen einen weiteren Vertreter der Überfamilie Heterakoidea, *Ascaridia galli* im Huhn, der zur Familie der Ascaridiidae gehört. Hier wirkt die Verbindung bei einer Dosierung von 2 mg/kg (16). Diese Substanzen aus der Verbindungsklasse der cyclischen Depsipeptide eigenen sich daher zur prophylaktischen Bekämpfung insbesondere der Histomoniasis.

Bevorzugt eingesetzte Depsipeptid-Anthelmintika sind 24-gliedrige Cyclodepsipeptide. Als solche seien genannt:
Verbindungen der Formel (IIa) 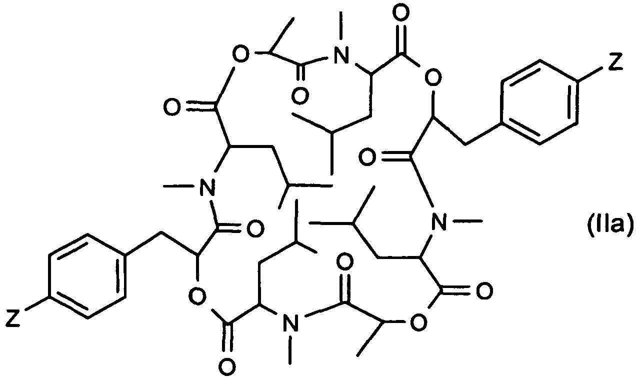 in welcher
Z für Wasserstoff, N-Morpholinyl, NH₂, Mono- oder Dimethylamino steht. Außerdem seien Verbindungen der folgenden Formel (IIb) genannt: 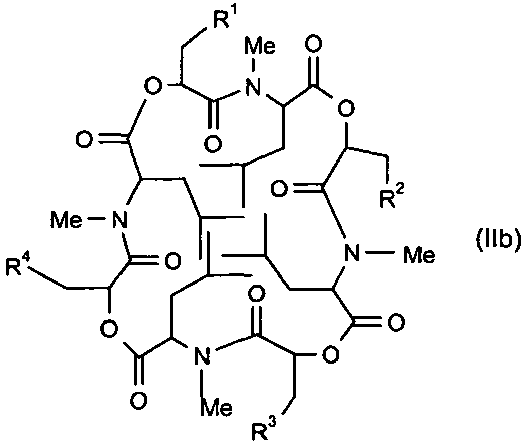 in welcher
R¹, R², R³, R⁴ unabhängig voneinander für Wasserstoff, C₁-C₁₀-Alkyl oder Aryl, insbesondere Phenyl stehen, die gegebenenfalls substituiert sind durch Hydroxy, C₁-C₁₀-Alkoxy oder Halogen.

Die Verbindungen der allgemeinen Formel (IIb) sind bekannt und können nach den in EP-A-382 173, DE-A 4 317 432, DE-A 4 317 457, DE-A 4 317 458, EP-A-634 408, EP-A-718 293, EP-A-872 481, EP-A-685 469, EP-A-626 375, EP-A-664 297, EP-A-669 343, EP-A-787 141, EP-A-865 498, EP-A-903 347 beschriebenen Verfahren erhalten werden.

Zu den cyclischen Depsipeptiden mit 24 Ringatomen zählen auch Verbindungen der allgemeinen Formel (IIc) 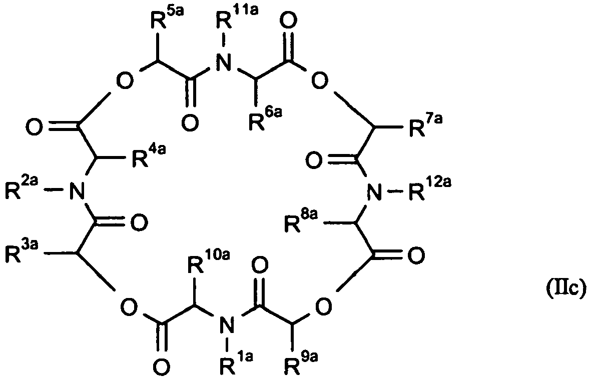 in welcher
R^{1a}, R^{2a}, R^{11a} und R^{12a} unabhängig voneinander für C₁₋₈-Alkyl, C₁₋₈-Halogenalkyl, C₃₋₆-Cycloalkyl, Aralkyl, Aryl stehen,
R^{3a}, R^{5a}, R^{7a}, R^{9a} unabhängig voneinander für Wasserstoff oder geradkettiges oder verzweigtes C₁₋₈-Alkyl steht, das gegebenenfalls durch Hydroxy, C₁₋₄-Alkoxy, Carboxy, 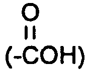 , Carboxamid, 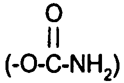 , Imidazolyl, Indolyl, Guanidino, -SH oder C₁₋₄-Alkylthio substituiert sein kann und ferner für Aryl oder Aralkyl die durch Halogen, Hydroxy, C₁₋₄-Alkyl, C₁₋₄-Alkoxy substituiert sein können, steht,
R^{4a}, R^{6a}, R^{8a}, R^{10a} unabhängig voneinander für Wasserstoff, geradkettiges C₁₋₅-Alkyl, C₂₋₆-Alkenyl, C₃₋₇-Cycloalkyl, die gegebenenfalls durch Hydroxy, C₁₋₄-Alkoxy, Carboxy, Carboxamid, Imidazolyl, Indolyl, Guanidino, SH oder C₁₋₄-Alkylthio substituiert sein können, sowie für Aryl oder Aralkyl die durch Halogen, Hydroxy, C₁₋₄-Alkyl, C₁₋₄-Alkoxy substituiert sein können, stehen sowie deren optische Isomere und Racemate.

Bevorzugte Verbindungen der Formel (IIc) sind solche, worin R^{1a}, R^{2a}, R^{11a} und R^{12a} unabhängig voneinander für Methyl, Ethyl, Propyl, Isopropyl, n-, s-, t-Butyl oder Phenyl, das gegebenenfalls substituiert ist durch Halogen, C₁₋₄-Alkyl, OH, C₁₋₄-Alkoxy, sowie für Benzyl oder Phenylethyl stehen, die gegebenenfalls durch die bei Phenyl angegebenen Reste substituiert sein können;
R^{3a} bis R^{10a} die oben angegebene Bedeutung haben.

Besonders bevorzugte Verbindungen der Formel (IIc), sind solche, worin R^{1a}, R^{2a}, R^{11a} und R^{12a} unabhängig voneinander für Methyl, Ethyl, Propyl, Isopropyl oder n-, s-, t-Butyl stehen,
R^{3a}, R⁵⁸, R^{7a}, R^{9a} für Wasserstoff, geradkettiges oder verzweigtes C₁₋₈-Alkyl, insbesondere Methyl, Ethyl, Propyl, i-Propyl, n-, s-, t-Butyl, die gegebenenfalls durch C₁₋₄-Alkoxy, insbesondere Methoxy, Ethoxy, Imidazolyl, Indolyl oder C₁₋₄-Alkylthio, insbesondere Methylthio, Ethylthio substituiert sein können, ferner für Phenyl, Benzyl oder Phenethyl stehen, die gegebenenfalls durch Halogen insbesondere Chlor substituiert sein können.
R^{4a}, R^{6a}, R^{8a}, R^{10a} unabhängig voneinander für Wasserstoff, Methyl, Ethyl, n-Propyl, n-Butyl, Vinyl, Cyclohexyl, die gegebenenfalls durch Methoxy, Ethoxy, Imidazolyl, Indolyl, Methylthio, Ethylthio substituiert sein können, sowie für Isopropyl, s-Butyl ferner für gegebenenfalls halogensubstituiertes Phenyl, Benzyl oder Phenylethyl stehen.

Die Verbindungen der Formel (IIc) können ebenfalls nach den in EP-A-382 173, DE-A 4 317 432, DE-A 4 317 457, DE-A 4 317 458, EP-A-634 408, EP-A-718 293, EP-A-872 481, EP-A-685 469, EP-A-626 375, EP-A-664 297, EP-A-669 343, EP-A-787 141, EP-A-865 498, EP-A-903 347 beschriebenen Verfahren erhalten werden.

Als ganz besonders bevorzugtes Depsipeptid sei die aus EP-OS 382 173 bekannte Verbindung PF 1022, es handelt sich um die Verbindung der Formel (IIa), worin beide Substituenten Z für Wasserstoff stehen. PF 1022 hat daher die folgende Formel (IId):

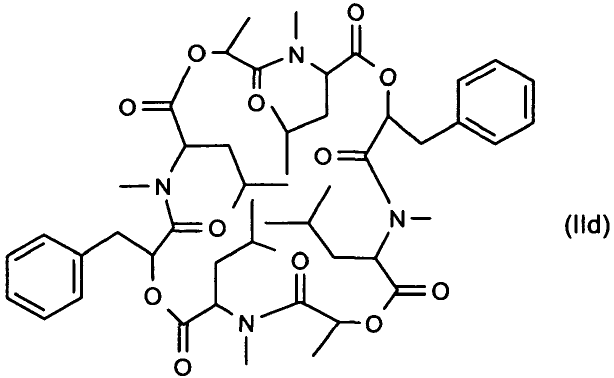

Weitere bevorzugte Depsipeptide sind Verbindungen, die aus der PCT-Anmeldung WO 93/19053 bekannt sind, und zwar Verbindungen der Formel (IIa), in welcher
Z für N-Morpholinyl, NH₂, Mono- oder Dimethylamino steht.

Von diesen Verbindungen ganz besonders bevorzugt ist das Depsipeptid Emodepside (PF 1022-221). Es handelt sich um die Verbindung der Formel (IIa), worin beide Reste Z für den Morpholinylrest stehen. Der INN Emodepside steht für die Verbindung mit dem systematischen Namen: Cyclo[(*R*)-lactoyl-*N*-methyl-L-leucyl-(*R*)-3-(*p*-morpholinophenyl)lactoyl-*N*-methyl-L-leucyl-(*R*)-lactoyl-*N-*methyl-L-leucyl-(*R*)-3-(*p*-morpholinophenyl)lactoyl-*N*-methyl-L-leucyl. Emodepside ist in WO 93/19053 beschrieben und hat die folgende Formel:

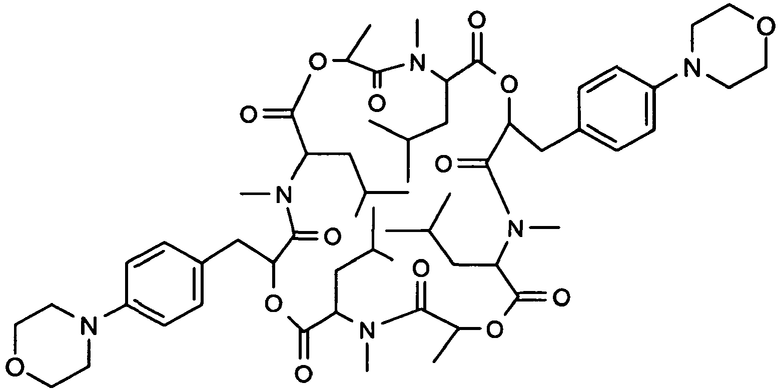

Die vorstehend genannten zur Kombination geeigneten Wirkstoffe können je nach Struktur in stereoisomeren Formen oder als Stereoisomerengemische vorliegen, z.B. als Enantiomere oder Racemate. Sowohl die Stereoisomerengemische als auch die reinen Stereoisomeren können erfindungsgemäß verwendet werden.

Weiterhin können gegebenenfalls verwendet werden: Salze der Wirkstoffe mit pharmazeutisch annehmbaren Säuren oder Basen und auch Solvate, insbesondere Hydrate, der Wirkstoffe oder ihrer Salze.

Die Anwendung in Kombination bedeutet entweder, dass Nifurtimox und der zweite Wirkstoff, insbesondere ein Cyclodepsipeptid, getrennt oder zeitlich abgestuft angewendet werden können. In diesem Fall sind Nifurtimox und der zweite Wirkstoff jeweils als gesonderte Arzneimittel formuliert.

Auch denkbar ist die gleichzeitige Anwendung. Gemäß einer für diesen Fall geeigneten Ausführungsform sind die Wirkstoffe der Kombination gemeinsam in einem Mittel formuliert. Anwendungsfertige Zubereitungen enthalten den jeweiligen Wirkstoff üblicherweise in Konzentrationen von 10 ppm bis 20 Gew.-%, bevorzugt von 0,1 bis 10 Gew.-%.

Zubereitungen die vor Anwendung verdünnt werden, enthalten den jeweiligen Wirkstoff in Konzentrationen von 0,5 bis 90 Gew.-%, bevorzugt von 5 bis 50 Gew.-%. In konzentrierten Lösungen zur Eindosierung ins Trinkwasser liegt der jeweilige Wirkstoff beispielsweise in Konzentrationen von 0,5 bis 20 Gew.-%, vorzugsweise 1 bis 15 Gew.-%, besonders bevorzugt 2 bis 10 Gew.-% vor.

Im Allgemeinen hat es sich als vorteilhaft erwiesen, Mengen von etwa 0,05 bis etwa 200 mg, bevorzugt 0,1 bis 100 mg, Wirkstoff je kg Körpergewicht pro Tag zur Erzielung wirksamer Ergebnisse zu verabreichen.

In der Mischung mit anderen Coccidiosemitteln oder Polyetherantibiotika liegen die erfindungsgemäßen Wirkstoffe in der Regel im Gewichtsverhältnis 1 zu 0,01 - 50 bis 1 zu 1 - 50 vor.

Die Wirkstoffe können auch zusammen mit dem Futter oder Trinkwasser der Tiere verabreicht werden.

Futter- und Nahrungsmittel enthalten 0,005 bis 1000 ppm, vorzugsweise 0,05 bis 500 ppm des Wirkstoffs in Kombination mit einem geeigneten essbaren Material.

Ein solches Futter- und Nahrungsmittel kann sowohl für Heilzwecke als auch für prophylaktische Zwecke verwendet werden.

Die Herstellung eines solchen Futter- oder Nahrungsmittels erfolgt durch Mischen eines Konzentrats oder einer Vormischung, die 0,5 bis 30 %, vorzugsweise 1 bis 20 Gew.-% eines Wirkstoffs in Mischung mit einem essbaren organischen oder anorganischen Träger enthält mit üblichen Futtermitteln. Essbare Träger sind z.B. Maismehl oder Mais- und Sojabohnenmehl oder Mineralsalze, die vorzugsweise eine geringe Menge eines essbaren Staubverhütungsöls, z.B. Maisöl oder Sojaöl, enthalten. Die hierbei erhaltene Vormischung kann dann dem vollständigen Futtermittel vor seiner Verfütterung an die Tiere zugesetzt werden.

Beispielhaft sei der Einsatz bei der Histomoniasis beschrieben:
Für die Heilung und Prophylaxe der Histomoniasis bei Geflügel, insbesondere bei Hühnern, Enten, Gänsen oder Truthähnen, werden 0,005 bis 1000 ppm, vorzugsweise 0,05 bis 500 ppm eines Wirkstoffs mit einem geeigneten eßbaren Material, z.B. einem nahrhaften Futtermittel, gemischt. Falls gewünscht, können diese Mengen erhöht werden, besonders wenn der Wirkstoff vom Empfänger gut vertragen wird. Entsprechend kann die Verabreichung über das Trinkwasser erfolgen.

Trotzdem kann es zeitweilig notwendig sein, von den genannten Mengen abzuweichen, insbesondere in Abhängigkeit vom Körpergewicht des Versuchstieres oder der Art der Verabreichungsmethode, aber auch wegen der Tiergattung und seiner individuellen Reaktion auf den Wirkstoff oder der Art der Formulierung und der Zeit oder dem Abstand, zu dem er verabreicht wird. So kann es in gewissen Fällen genügen, mit weniger als der vorstehend genannten Mindestmenge auszukommen, während in anderen Fällen die genannte obere Grenze überschritten werden muss. Bei der Verabreichung größerer Mengen kann es zweckmäßig sein, diese im Verlauf des Tages in mehrere Einzeldarreichungen zu unterteilen.

Die Wirksamkeit der erfindungsgemäßen Verbindungen lässt sich z.B. in Käfigversuchen mit folgender Versuchsanordnung belegen, bei der die Tiere mit dem jeweiligen Wirkstoff behandelt werden.

Ein wirkstoffhaltiges Futter wird so zubereitet, dass die erforderliche Menge Wirkstoff mit einem nährstoffmäßig ausgeglichenen Tierfutter, z.B. mit dem unten angegebenen Kükenfutter, gründlich vermischt wird.

Wenn ein Konzentrat oder eine Vormischung zubereitet werden soll, die schließlich im Futter auf die im Versuch genannten Werte verdünnt werden soll, werden im Allgemeinen etwa 1 bis 30 %, vorzugsweise etwa 10 bis 20 Gew.-% Wirkstoff mit einem essbaren organischen oder anorganischen Träger, z.B. Mais- und Sojamehl oder Mineralsalzen, die eine kleine Menge eines essbaren Entstäubungsöls, z.B. Maisöl oder Sojabohnenöl enthalten, vermischt. Die so erhaltene Vormischung kann dann dem vollständigen Geflügelfutter vor der Verabreichung zugegeben werden.

Als Beispiel für die Verwendung der erfindungsgemäßen Stoffe im Geflügelfutter kommt die folgende Zusammensetzung in Frage.

| | |
|---|---|
| 52,00 % | Futtergetreideschrot, und zwar: 40 % Mais, 12 % Weizen |
| 17,00 % | Sojaschrot extr. |
| 5,00 % | Maisklebefutter |
| 5,00 % | Weizenfuttermehl |
| 3,00 % | Fischmehl |
| 3,00 % | Mineralstoffmischung |
| 3,00 % | Luzernegrasgrünmehl |
| 2,50 % | Vitaminvormischung |
| 2,00 % | Weizenkeime, zerkleinert |
| 2,00 % | Sojaöl |
| 2,00 % | Fleischknochenmehl |
| 1,50 % | Molkenpulver |
| 1,00 % | Melasse |
| 1,00 % | Bierhefe, gebunden an Biertreber |
| 100,00 % | |

Ein solches Futter enthält 18 % Rohprotein, 5 % Rohfaser, 1 % Ca, 0,7 % P sowie je kg 1200 i.E. Vitamin A, 1200 i.E. Vitamin D3, 10 mg Vitamin E, 20 mg Zinkbacitracin.

### Literatur:

1. Raether W., Hänel H. (2003): Nitroheterocyclic drugs with broad spectrum activity Parasitol Res. 90:S19-S39.
2. Harder A., Greif G., Haberkom A. (2001a): Chemotherapeutic approoaches to protozoa: Haemosporina - current level of knowledge and outlook.
3. Harder A., Greif G., Haberkorn A. (2001b): Chemotherapeutic approaches to protozoa: Giardia, Trichomonas and Entamoeba - current level of knowledge and outlook.
4. Greif G., Harder A., Haberkorn A. (2001): Chemotherapeutic approaches to protozoa: Caccidiae - current level of knowledge and outlook.
5. Harder A., Greif G., Haberkom A. (2001c): Chemotherapeutic approaches to protozoa: Kinetoplastida - current level of knowledge and outlook. Parasitol Res 87:778-780.
6. Kulda J. (1999): Trichomonas, hydrogenosomes and drug resistance. International Journal for Parasitology 29:199-212
7. McDougald L.R. (2005): Blackhead Disease (Histomoniasis) in Poultry: A critical Review. Avian Diseases 49(4):462-476.
8. McDougald, L.R., Hu, J. (2001): Blackhead Disease (Histomonas meleagridis) aggravated in broiler chickens by cncurrent infection with cecal coccidiosis (Eimeria tenella). Avian Diseases 45:307-312
9. Hegngi F.N., Doerr J., Cummings T.S., Schwartz R.D., Saunders G., Zajac A., Larsen C.T., Piierson F.W.(1999): The effectiveness of benzimidazole derivatives fort the treatment and prevention of histomonosis (blachhead) in turkeys. Veterinary Parasitology 81:29-37.
10. Hegngi, F.N., Doeerr, J., Cummings, T.S., Schwartz, R.D., Saunders, G., Zajac, A., Larsen, C.T., Pierson, F.W. (1999): The effectiveness of benzimidazole derivatives for the treatment and prevention of histomonosis (bleackhead) in turkeys. Veterinary Parasitology 81:29-37.
11. von Samson-Himmelstjema G., Harder A., Schnieder T., Kalbe J., Mencke N. (2000): In vivo activities of the new anthelmintic depsipeptide PF1022A. Parasitol. Res. 86 : 194-199
12. Mehlhom H., Nicolay F., Harder A., von Samson-Himmelstjerna A. (2000): Synergistic action of Bay 44-4400 and piperazine on nematodes of the mouse in vitro and in vivo : a light and transmission electron microscopic study. Parasitol. Res. 86 : 982-992
13. Harder A., Schmitt-Wrede H.-P., Krücken J., Marinovski P., Wunderlich F., Willson J., Amliwala K., Holden-Dye L., Walker R.(2003): Cyclooctadepsipeptides - an anthelmintically active class of compounds exhibiting a novel mode of action. Int. J. Antimicrobial Agents 22 : 318-331
14. Mehlhorn H., Schmahl G., Frese M., Mevissen I., Harder A., Krieger K. (2005): Effects of a combination emodepside and praziquantel on parasites of reptiles and rodents. Parasitol. Res. 97 Suppl 1 : S65-S69
15. Bernt U., Junkersdorf B., Londershausen M., Harder A., Schierenberg E. (1998): Effects of anthelmintics with different modes of action on the behaviour and development of Caenorhabditis elegans. Fundam. Appl. Nematol. 21 : 251-263
16. Sasaki T, Takagi M, Yaguchi T, Miyadoh S, Okada T, Koyama S (1992) A new anthelmintic cyclodepsipeptide, PF1022A. Journal of Antibiotics 45 : 692-697

### Beispiel

### Medikierte Futter

Durch Einmischen von pulverförmigem Nifurtimox in Konzentrationen von 50, 100, 200 und 400 ppm in die unten angegebene Futtermischung können medikierte Futter hergestellt werden.

| | |
|---|---|
| 52,00 % | Futtergetreideschrot, und zwar: 40 % Mais, 12 % Weizen |
| 17,00 % | Sojaschrot extr. |
| 5,00 % | Maisklebefutter |
| 5,00 % | Weizenfuttermehl |
| 3,00 % | Fischmehl |
| 3,00 % | Mineralstoffmischung |
| 3,00 % | Luzernegrasgrünmehl |
| 2,50 % | Vitaminvormischung |
| 2,00 % | Weizenkeime, zerkleinert |
| 2,00 % | Sojaöl |
| 2,00 % | Fleischknochenmehl |
| 1,50 % | Molkenpulver |
| 1,00 % | Melasse |
| 1,00 % | Bierhefe, gebunden an Biertreber |
| 100,00 % | |

### Tabletten

Nifurtimox-Tabletten sind bekannt und z. B. unter dem Handelsnamen Lampit® als Arzneimittel erhältlich.

### A. Biologisches Beispiel: Käfigversuche Histomonadenwirksamkeit in Puten

Histomonaden-freie 10 Tage alte männliche Puten erhalten von Tag -4 (= 4 Tage vor der Infektion) bis Tag 14 Nifurtimox oder die Vergleichsverbindung Nitarson in der in "ppm" angegebenen Konzentration mit dem Futter. Die Infektion erfolgt am Tag 0. In jedem Gruppenkäfig werden 10 Tiere gehalten. Je Dosierung werden 1 bis 3 derartige Gruppen eingesetzt.

Die Infektion erfolgt mit einem Histomonaden-Feldstamm, der im Labor passagiert und in flüssigem Stickstoff gelagert wird. Am Tag 0 werden jeweils 5 Tiere eines Käfigs (außer der nicht infizierten Kontrolle) intracloakal mit 250.000 Histomonaden in jeweils 1 ml Dwyer"s Medium infiziert (= direkte Infektion). Diese infizierten Tiere scheiden nach wenigen Tagen neue Histomonaden aus und übertragen damit den Erreger auf die restlichen 5 Tiere des Käfigs (= indirekte Infektion).

Für die Beurteilung der Wirksamkeit werden die Kriterien nach Mc Dougald und Hu 2001 (7) berücksichtigt:
- die infektionsbedingte Sterberate
- die Gewichtszunahme von Versuchsbeginn bis Versuchsende
- Futteraufnahme
- Futterverwertung
- Makroskopische Beurteilung der infektionsabhängigen Läsionen in den Blinddärmen (Caecum) und in der Leber. Bei dieser Bewertung bedeutet "Score 0" = keine Läsionen und "Score 4" = schwerwiegende Läsionen.

**Tabelle 1: Studien Design 210 Tiere**

| Gruppe | Ccode | Behandlung | | | Infektion |
|---|---|---|---|---|---|
| | | Verbindung | Konzentration | Tag | |
| Uninf. | D | unbehandelt | - | - | - |
| Inf. contr. | F | unbehandelt | - | - | + |
| 30 ppm | E | Nifurtimox | 30 ppm | -4 to +14 | + |
| 60 ppm | B | Nifurtimox | 60 ppm | -4 to +14 | + |
| 120 ppm | C | Nifurtimox | 120 ppm | -4 to +14 | + |
| 200 ppm | G | Nifurtimox | 200 ppm | -4 to +14 | + |
| Nita. | A | Nitarsone | 187.5 ppm | -4 to +14 | + |

**Tabelle 2: Studien Design 210 Tiere**

| **Gruppe** | **Code** | **Behandlung** | | | **Infektion** |
|---|---|---|---|---|---|
| | | Verbindung | Konzentration | Tag | |
| Uninf. | G | unbehandelt | - | - | - |
| Inf. contr. | A | unbehandelt | - | - | + |
| 100 ppm | D | Nifurtimox | 100 ppm | -4 to +14 | + |
| 200 ppm | F | Nifurtimox | 200 ppm | -4 to +14 | + |
| 300 ppm | B | Nifurtimox | 300 ppm | -4 to +14 | + |
| 400 ppm | C | Nifurtimox | 400 ppm | -4 to +14 | + |
| Nita. | E | Nitarsone | 187.5 ppm | -4 to +14 | + |

**Tabelle 3: Histomoniasis verursachte Mortalität**

| Behandlung | Mortalität | | Behandlung | Mortalität |
|---|---|---|---|---|
| inf. contr. | 7 | | inf. contr. | 11 |
| 30 ppm | 5 | | 100 ppm | 13 |
| 60 ppm | 6 | | 200 ppm | 4 |
| 120 ppm | 5 | | 300 ppm | 2 |
| 200 ppm | 4 | | 400 ppm | 0 |
| Nita. | 1 | | Nita. | 2 |

**Tabelle 4: Mittlere Gewichtszunahme (g) zwischen Tag 0 und dem Zeitpunkt des Todes**

| **Behandlung** | **Direkte Infektion** | | | **Behandlung** | **Direkte Infektion** | |
|---|---|---|---|---|---|---|
| | **nein** | **ja** | | | **nein** | **ja** |
| Uninf. | 791 | | | uninf. | 819 | |
| Inf. contr. | 741 | 197 | | inf. contr. | 666 | 259 |
| 30 ppm | 730 | 235 | | 100 ppm | 581 | 135 |
| 60 ppm | 838 | 304 | | 200 ppm | 525 | 270 |
| 120 ppm | 744 | 182 | | 300 ppm | 750 | 450 |
| 200 ppm | 744 | 264 | | 400 ppm | 832 | 691 |
| Nita. | 782 | 541 | | Nita. | 725 | 426 |

**Tabelle 5: Mittlerer Futterverbrauch zwischen Tag 0 und Tag 14**

| Behandlung | Futterverbrauch (kg) | | Behandlung | Futterverbrauch (kg) |
|---|---|---|---|---|
| Nicht inf. Kontrolle | 12.01 | | Nicht inf. Kontrolle | 12.44 |
| Infizierte Kontrolle | 8.84 | | Infizierte Kontrolle | 9.56 |
| 30 ppm | 9.00 | | 100 ppm | 6.69 |
| 60 ppm | 9.68 | | 200 ppm | 9.14 |
| 120 ppm | 8.70 | | 300 ppm | 10.40 |
| 200 ppm | 9.59 | | 400 ppm | 11.79 |
| Nita. | 11.64 | | Nita. | 9.82 |

**Tabelle 6: AMittlere Futterverwertung zwischen Tag 0 und Tag 14**

| Behandlung | Futterverwertung | | Behandlung | Futterverwertung |
|---|---|---|---|---|
| Nicht inf. Kontrolle | 1.52 | | Nicht inf. Kontrolle | 1.63 |
| Infizierte Kontrolle | 1.89 | | Infizierte Kontrolle | 2.10 |
| 30 ppm | 1.88 | | 100 ppm | 2.32 |
| 60 ppm | 1.70 | | 200 ppm | 2.33 |
| 120 ppm | 1.88 | | 300 ppm | 1.83 |
| 200 ppm | 1.91 | | 400 ppm | 1.55 |
| Nita. | 1.77 | | Nita. | 1.79 |

**Tabelle 7: Mittlere Läsionen im Blinddarm**

| **Behandlung** | **Direkte Infektion** | | | **Behandlung** | **Direkte Infektion** | |
|---|---|---|---|---|---|---|
| | **nein** | **ja** | | | **nein** | **ja** |
| Nicht inf. Kontrolle | 0.03 | | | Nicht inf. Kontrolle | 0.00 | |
| Inf. Kontrolle | 0.29 | 3.93 | | Inf. Kontrolle | 0.93 | 2.93 |
| 30 ppm | 0.29 | 3.73 | | 100 ppm | 1.08 | 3.79 |
| 60 ppm | 0.00 | 3.53 | | 200 ppm | 1.80 | 3.67 |
| 120 ppm | 0.53 | 4.00 | | 300 ppm | 0.79 | 2.53 |
| 200 ppm | 0.47 | 3.80 | | 400 ppm | 0.27 | 1.20 |
| Nita. | 0.00 | 2.26 | | Nita. | 0.29 | 3.07 |

**Tabelle 8: Infektionsabhängige Läsionen in der Leber**

| **Behandlung** | **Direkte Infektion** | | | **Behandlung** | **Direkte Infektion** | |
|---|---|---|---|---|---|---|
| | **no** | **yes** | | | **no** | **yes** |
| Uninf. | 0.00 | | | uninf. | 0.00 | |
| Inf. contr. | 0.29 | 3.47 | | inf. contr. | 0.14 | 1.80 |
| 30 ppm | 0.29 | 2.53 | | 100 ppm | 0.58 | 2.00 |
| 60 ppm | 0.00 | 2.53 | | 200 ppm | 0.73 | 1.33 |
| 120 ppm | 0.27 | 1.80 | | 300 ppm | 0.07 | 0.40 |
| 200 ppm | 0.13 | 1.33 | | 400 ppm | 0.00 | 0.00 |
| Nita. | 0.00 | 0.13 | | Nita. | 0.21 | 0.79 |

### B. Biologisches Beispiel: Anthelminthische Eigenschaften der cyclischen Oktadepsipeptide

Männliche Mäuse (Stamm Bor CFW, Körpergewicht zwischen 25 bis 30g) werden in Makrolonkäfigen gehalten (3 Tiere/Käfig) und werden mit Wasser und SNIFF Rattenfutter (10 mm-Pellets) ad libitum versorgt. Mäuse werden infiziert mit *Heterakis spumosa* durch orale Applikation von 90 embryonierten Eiern. Die Eier wurden aus weiblichen Würmern gewonnen, die aus dem Kolon der Mäuse 40 Tage nach der Infektion isoliert wurden. Die Eier wurden weitere 3 Wochen bei 37°C inkubiert. 35 Tage nach Infektion werden die Mäuse mit der entsprechenden Dosis von PF1022A oder Emodepsid an vier aufeinander folgenden Tagen behandelt. PF1022A wird in Cremophor EL suspendiert. Die Mäuse werden am Tag 7 nach Behandlung getötet, der Dünndarm/Caecum/Kolon-Bereich entnommen und die Würmer makroskopisch gezählte. Das Verhältnis der Zahl der ausgetriebenen Würmer in Prozent der Gesamtzahl der Würmer in unbehandelten, infizierten Kontrolltieren wird als Maß für die anthelminthische Wirksamkeit definiert.

Die Experimente am Huhn werden in (14) und der darin zitierten Literatur beschrieben.

**Tabelle 9: Anthelminthische Aktivität von PF1022A und Emodepsid gegen Nematoden aus der Überfamilie der Heterakoidea**

| **Nematode** | **Dosierung mit voller Wirksamkeit** |
|---|---|
| PF1022A | |
| *Heterakis spumosa* (Maus) | 50 mg/kg |
| *Ascaridia galli* (Huhn) | 2 mg/kg |
| Emodepsid | |
| *Heterakis spumosa* (Maus) | 1-10 mg/kg |

## Patentansprüche

1. Die Verwendung von Nifurtimox zur Herstellung von Arzneimitteln zur Behandlung von durch Trichomonadida hervorgerufenen Krankheiten.

2. Verwendung gemäß Anspruch 1 zur Behandlung von durch Histomonaden hervorgerufenen Krankheiten.

3. Verwendung gemäß Anspruch 2 zur Behandlung von durch *Histomonas meleagridis* hervorgerufenen Krankheiten.

4. Verwendung gemäß Anspruch 1 zur Behandlung von durch Trichomonaden hervorgerufenen Krankheiten.

5. Verwendung gemäß Anspruch 1 zur Behandlung von durch Tritrichomonaden hervorgerufenen Krankheiten.

6. Nifurtimox zur Verwendung zur Behandlung der von durch Trichomonadida hervorgerufenen Krankheiten.

7. Nifurtimox zur Verwendung gemäß Anspruch 6 zur Behandlung von durch Histomonaden hervorgerufenen Krankheiten.

8. Nifurtimox zur Verwendung gemäß Anspruch 7 zur Behandlung von durch *Histomonas meleagridis* hervorgerufenen Krankheiten.

9. Nifurtimox zur Verwendung gemäß Anspruch 6 zur Behandlung von durch Trichomonaden hervorgerufenen Krankheiten.

10. Nifurtimox zur Verwendung gemäß Anspruch 6 zur Behandlung von durch Tritrichomonaden hervorgerufenen Krankheiten.

11. Nifurtimox zur Verwendung gemäß einem der Ansprüche 6 bis 10 bei Geflügel.

12. Nifurtimox zur Verwendung gemäß Anspruch 11 bei Puten.

## Claims

1. Use of nifurtimox for the preparation of pharmaceuticals for the treatment of diseases caused by Trichomonadida.

2. Use according to Claim 1 for the treatment of diseases caused by histomonads.

3. Use according to Claim 2 for the treatment of diseases caused by *Histomonas meleagridis.*

4. Use according to Claim 1 for the treatment of diseases caused by trichomonads.

5. Use according to Claim 1 for the treatment of diseases caused by tritrichomonads.

6. Nifurtimox for use in the treatment of the diseases caused by Trichomonadida.

7. Nifurtimox for use according to Claim 6 in the treatment of diseases caused by histomonads.

8. Nifurtimox for use according to Claim 7 in the treatment of diseases caused by *Histomonas meleagridis.*

9. Nifurtimox for use according to Claim 6 in the treatment of diseases caused by trichomonads.

10. Nifurtimox for use according to Claim 6 in the treatment of diseases caused by tritrichomonads.

11. Nifurtimox for use according to one of Claims 6 to 10 in poultry.

12. Nifurtimox for use according to Claim 11 in turkeys.

## Revendications

1. Utilisation de nifurtimox pour fabriquer des médicaments destinés au traitement de maladies provoquées par Trichomonadida.

2. Utilisation selon la revendication 1 pour le traitement de maladies provoquées par des histomonades.

3. Utilisation selon la revendication 2 pour le traitement de maladies provoquées par *Histomonas meleagridis.*

4. Utilisation selon la revendication 1 pour le traitement de maladies provoquées par des trichomonades.

5. Utilisation selon la revendication 1 pour le traitement de maladies provoquées par des tritrichomonades.

6. Nifurtimox pour une utilisation pour le traitement des maladies provoquées par Trichomonadida.

7. Nifurtimox pour utilisation selon la revendication 6 pour le traitement de maladies provoquées par des histomonades.

8. Nifurtimox pour utilisation selon la revendication 7 pour le traitement de maladies provoquées par *Histomonas meleagridis.*

9. Nifurtimox pour utilisation selon la revendication 6 pour le traitement de maladies provoquées par des trichomonades.

10. Nifurtimox pour utilisation selon la revendication 6 pour le traitement de maladies provoquées par des tritrichomonades.

11. Nifurtimox pour utilisation selon l'une des revendications 6 à 10 sur les volailles.

12. Nifurtimox pour utilisation selon la revendication 11 sur les dindes.
